# EUROPEAN PATENT SPECIFICATION

(11) **EP 2 173 694 B1**
(45) Date of publication and mention of the grant of the patent: **23.11.2016**
(21) Application number: 08772376.3
(22) Date of filing: 02.07.2008
(51) Int. Cl.: C07C 27/06, C07C 29/00, B01J 21/04

(54) **METHODS FOR PRODUCING ALCOHOLS FROM SYNGAS**
VERFAHREN ZUR HERSTELLUNG VON ALKOHOLEN AUS SYNTHESEGAS
PROCÉDÉS POUR PRODUIRE DES ALCOOLS À PARTIR D'UN GAZ DE SYNTHÈSE

(30) Priority: 09.07.2007 US 948650 P; 09.07.2007 US 948657 P; 01.07.2008 US 166203; 01.07.2008 US 166212
(43) Date of publication of application: 14.04.2010
(73) Proprietor: Albemarle Corporation, Baton Rouge, LA 70801-1765 (US)
(72) Inventor: KLEPPER, Robert, E., Arvada, Colorado 80003 (US); GEERTSEMA, Arie, Westminster, Colorado 80031 (US); ROBOTA, Heinz, Juergen, Arvada, Colorado 80005 (US); STITES, Ronald, C., Brighton, Colorado 80601 (US); RIDLEY, Richard, Loveland, Colorado 80538 (US); TIRMIZI, Shakeel, H., Matawa, New Jersey 07747 (US); FERRARO, Francis, M., Westminster, Colorado 80234 (US)
(74) Representative: van Heesch, Helmut Werner
(86) International application number: PCT/US2008/069071
(87) International publication number: WO 2009/009389

(56) References cited:
- US-A- 4 725 626
- US-A1- 2005 107 482
- US-A1- 2006 009 537
- US-B2- 6 762 332
- US-B2- 7 169 821

## Description

### FIELD OF THE INVENTION

The present invention generally relates to processes for the conversion of carbonaceous feedstocks, such as cellulosic biomass, into synthesis gas, and to processes for the conversion of synthesis gas to products such as alcohols (e.g., ethanol).

### BACKGROUND OF THE INVENTION

Ethanol and alcohol mixtures including ethanol may be used as fuels and fuel additives in place of petroleum-based products such as gasoline. Such use of alcohols can reduce the need to import petroleum. In addition, the substitution of alcohols for petroleum-based fuels and fuel additives can be particularly environmentally friendly when the alcohols are produced from feedstocks other than fossil fuels.

One synthetic route to alcohols is through catalytic processes for the conversion of syngas to alcohols. Syngas (or synthesis gas) is a mixture of monoxide (CO) and hydrogen (H₂). Generally, syngas may be produced from any carbonaceous material. In particular, biomass such as, for example, agricultural wastes, forest products, grasses, and other cellulosic material may be converted to syngas.

There exist a variety of conversion technologies to turn these feedstocks into syngas. Conversion approaches can utilize a combination of one or more steps comprising gasification, pyrolysis, steam reforming, and/or partial oxidation of a carbonaceous material.

Since the 1920s it has been known that mixtures of methanol and other alcohols can be obtained by reacting syngas over certain catalysts (Forzatti et al., Cat. Rev.-Sci. and Eng. 33(1-2), 109-168, 1991). Fischer and Tropsch observed around the same time that hydrocarbon-synthesis catalysts produced linear alcohols as byproducts (Fischer and Tropsch, Brennst.-Chem. 7:97, 1926).

US 2005/0107482 describes a process for producing light olefins including as an intermediate step the production of methanol and as a subsequent intermediate step the production of ethanol using syngas and conversion and homologation catalysts.

However, improved methods and apparatus to convert syngas into alcohols, such as ethanol, are currently needed.

### SUMMARY OF THE INVENTION

The present invention provides a method of producing one or more C₂-C₄ alcohols, the method comprising:
(i) introducing syngas into a first reaction zone comprising at least a first catalyst;
(ii) converting a portion of up to 50% CO of said syngas to methanol with the first catalyst;
(iii) introducing syngas and methanol from the first reaction zone into a second reaction zone comprising at least a second catalyst; and
(iv) converting at least a portion of the syngas and methanol introduced into the second reaction zone with the second catalyst to produce a product stream comprising one or inore C₂-C₄ alcohols selected from ethanol, 1-propanol and 1-butanol.

The second reaction zone can be in the same reactor, or in a different reactor, than the first reaction zone.

The syngas introduced into the first reaction zone has an initial H₂/CO ratio of 1.5 to 2, conversion of syngas to methanol in the first reaction zone causes the syngas introduced into the second reaction zone to have a second H₂/CO ratio, and the second H₂/CO ratio provides an increased yield to one or more C₂-C₄ alcohols in the second reaction zone compared to that which would be provided by the initial H₂/CO ratio. The second H₂/CO ratio is lower than the initial H₂/CO ratio. The first catalyst and the second catalyst are different physical materials.

In some embodiments, the method further comprises introducing additional methanol into the second reaction zone and converting at least a portion of the additional methanol introduced into the second reaction zone to one or more C2-C₄ alcohols with the second catalyst. In certain embodiments, at least a portion of the additional methanol introduced into the second reaction zone was previously recovered from the product stream. In some methods, additional syngas (which is not unreacted syngas from the first reaction zone) is introduced into the second reaction zone. followed by converting at least a portion of the additional syngas introduced into the second reaction zone to one or more C₂-C₄ alcohols with the second catalyst. Syngas can be recovered from the product stream and recycled through at least one of the reaction zones.

The first catalyst can comprise a material selected from the group consisting of ZnO/Cr₂O₃, Cu/ZnO, Cu/ZnO/Al₂O₃, Cu/ZnO/Cr₂O₃, Cu/ThO₂, Co/S, Mo/S, Co/Mo/S, Ni/S, Ni/Mo/S, Ni/Co/Mo S, and any of the foregoing in combination with Mn and/or V. The first catalyst preferably includes a basic promoter.

The second catalyst can comprise a material selected from the group consisting of ZnO/Cr₂O₃, Cu/ZnO, Cu/ZnO/Al₂O₃, CuO/CoO, CuO/CoO/AlO₃, Co/S, Mo/S, Co/Mo/S, Rh/Ti/SiO₂, Rh/Mn/SiO₂, Rh/Ti/Fe/Ir/SiO₂, Rh/Mn/MCM-41, Ni/S, Ni/Mo/S, Ni/Co/Mo/S, and any of the foregoing in combination with Mn and/or V. The second catalyst preferably includes a basic promoter. The first catalyst and the second catalyst can, in some embodiments, have substantially the same initial composition.

In some embodiments, the first reaction zone is in a first reactor, the second reaction zone is in a second reactor, and an output stream of the first reactor comprises syngas introduced from the first reaction zone into the second reaction zone, further comprising separating from the output stream at least a portion of the methanol produced in the first reaction zone. The first reaction zone and second reaction zone can both be in a single reactor.

In certain embodiments, additional or recycled CO₂ can be introduced into the first reaction zone, wherein at least a portion of the CO₂ is reacted with H₂ present to produce CO₂-derived methanol. The CO₂-derived methanol can be converted, at least in part, to one or more C₂-C₄ alcohols (such as ethanol) in the second reaction zone.

### BRIEF DESCRIPTION OF THE FIGURES

FIG. 1 shows a process flow for producing methanol and ethanol from syngas using two reactors in sequence, according to one variation.
FIG. 2 shows a process flow for producing methanol and ethanol from syngas using two reaction zones in sequence in a single reactor, according to one variation.
FIG. 3 shows a process flow for producing methanol and ethanol from syngas using two reactors in sequence, with some or all of the methanol produced in the first reactor diverted from the second reactor, according to one variation.
FIG. 4 shows a process flow for producing methanol and ethanol from syngas using two reactors in sequence according to another variation.
FIG. 5 shows a process flow for producing methanol and ethanol from syngas using two reactors in sequence, with the first reactor producing methanol in high yield for conversion to ethanol in the second reactor, according to one variation.

### DETAILED DESCRIPTION OF EMBODIMENTS OF THE INVENTION

Certain embodiments of the present invention will now be further described in more detail, in a manner that enables the claimed invention so that a person of ordinary skill in this art can make and use the present invention.

As used herein, "C₁-C₄ alcohols" means one or more alcohols selected from methanol, ethanol, propanol and butanol. While some embodiments are described in relation to high selectivities to ethanol, the invention can also be practiced in a manner that gives high selectivities to methanol, propanol, and/or butanol, or certain combinations of selectivities to methanol, ethanol, propanol, and butanol. "C₂-C₄ alcohols" means one or more alcohols selected from ethanol, propanol, and butanol.

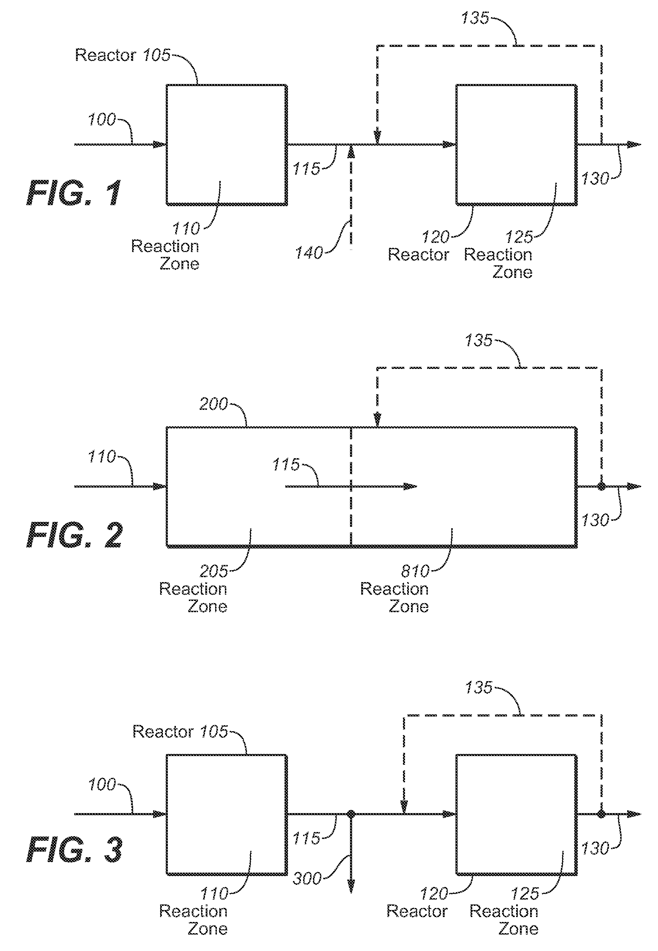

Methods and apparatus for producing C₁-C₄ alcohols from syngas are disclosed herein. In some variations of these methods and apparatus, syngas is catalytically converted to methanol in a first reaction zone, and residual syngas from the first reaction zone is then catalytically converted to ethanol in a second reaction zone. Referring to FIG. 1, for example, in one variation a syngas feedstream **100** is introduced into a first reactor **105** comprising a first reaction zone **110.** One or more catalysts in reaction zone **110** convert at least a portion of syngas feedstream **100** to methanol to provide an intermediate product stream **115** comprising at least a portion of the residual (unreacted) syngas from feedstream **100,** methanol, and, in some variations, higher alcohols and/or other reaction products.

At least a portion of intermediate product stream **115** is introduced into a second reactor **120** comprising a second reaction zone **125.** One or more catalysts in reaction zone **125** convert at least a portion of syngas from intermediate product stream **115** and/or at least a portion of methanol from intermediate product stream **115** to provide a product stream **130** comprising ethanol and, in some variations, methanol, higher alcohols, other reaction products, and/or unreacted syngas from intermediate product stream **115.**

Various components of product stream **130** such as, for example, methanol, ethanol, alcohol mixtures (e.g., methanol, ethanol, and/or higher alcohols), water, and unreacted syngas may be separated out and (optionally) purified by the methods described herein or by conventional methods. Such methods may include, for example, distillation and membrane separation processes as well as drying or purifying with molecular sieves.

Syngas feedstream **100** may be produced in any suitable manner known to one of ordinary skill in the art from any suitable feedstock. In some variations, syngas feedstream **100** is filtered, purified, or otherwise conditioned prior to being introduced into reactor **105.** For example, carbon dioxide, benzene, toluene, ethyl benzene, xylenes, sulfur compounds, metals, and/or other impurities or potential catalyst poisons may be removed from syngas feedstream **100** by conventional methods known to one of ordinary skill in the art.

Syngas feedstream **100** comprises H₂ and CO at a H₂/CO ratio having a value between 1.5 to 2.0. The H₂/CO ratio in feedstream **100** can, in some variations, affect the yield of methanol and other products in reactor **105.** The preferred H₂/CO ratio in such variations may depend on the catalyst or catalysts used in reactor **105** as well as on the operating conditions. Consequently, in some variations, the production and/or subsequent conditioning of syngas feedstream **100** is controlled to produce syngas having a H₂/CO ratio within a range desired to optimize, for example, production of methanol, ethanol, or both methanol and ethanol.

Syngas feedstream **100** may optionally be pressurized and/or heated by compressors and heaters (not shown) prior to entering reactor **105.** In some variations, syngas feedstream 100 enters reactor **105** at a temperature of 149°C (300°F) to 316°C (600°F) and at a pressure of 34.47 x 10⁵ Pag (500 psig) to 172.37 x 10⁵ Pag (2500 psig). In some embodiments, the temperature is between 149°C (300°F) to 204°C (400°F), 204°C (400°F) to 260°C (500°F), or 260°C (500°F) to 316°C (600°F). In some embodiments, the pressure is 34.47 x 10⁵ Pag (500 psig) to 68.95 x 10⁵ Pag (1000 psig), 68.95 x 10⁵ Pag (1000 psig) to 137.90 x 10⁵ Pag (2000 psig), or 137.90 x 10⁵ Pag (2000 psig) to 172.37 x 10⁵ Pag (2500 psig).

Reactor **105** may be any type of catalytic reactor suitable for the conversion of syngas to methanol, alcohol mixtures comprising methanol, higher alcohols, and/or other products. Reactor **105** may, for example, be any suitable fixed-bed reactor. In some variations, reactor **105** comprises tubes filled with one or more catalysts. Syngas passing through the tubes undergoes catalyzed reactions to form methanol and, in some variations, higher alcohols or other products. In some embodiments, catalysis occurs within pellets or in a homogeneous phase.

Reactor **105** may operate, for example, at temperatures of 204°C (400°F) to 371 °C (700°F) and at pressures of 34.47 x 10⁵ Pag (500 psig) to 172.37 x 10⁵ Pag (2500 psig). In some embodiments, the temperature is between 204°C (400°F) to 260°C (500°F), 260°C (500°F) to 316°C (600°F), or 316°C (600°F) to 371°C (700°F). In some embodiments, the pressure is 34.47 x 10⁵ Pag (500 psig) to 68.95 x 10⁵ Pag (1000 psig), 68.95 x 10⁵ Pag (1000 psig) to 137.90 x 10⁵ Pag (2000 psig), or 137.90 x 10⁵ Pag (2000 psig) to 172.37 x 10⁵ Pag (2500 psig).

In some embodiments, conditions effective for producing alcohols from syngas include average reactor residence times from 0.1-10 seconds, preferably 0.5-2 seconds. "Average reactor residence time" is the mean of the residence-time distribution of the reactor contents under actual operating conditions. Catalyst contact times can also be calculated by a skilled artisan and these times will typically also be in the range of 0.1-10 seconds, although it will be appreciated that it is certainly possible to operate at shorter or longer times.

The reactor for converting syngas into alcohols can be engineered and operated in a wide variety of ways. The reactor operation can be continuous, semicontinuous, or batch. Operation that is substantially continuous and at steady state is preferable. The flow pattern can be substantially plug flow, substantially well-mixed, or a flow pattern between these extremes. The flow direction can be vertical-upflow, vertical-downflow, or horizontal. A vertical configuration can be preferable.

The "reactor" can actually be a series or network of several reactors in various arrangements. For example, in some variations, the reactor comprises a large number of tubes filled with one or more catalysts.

Any suitable catalyst or combination of catalysts may be used in reactor 105 to catalyze reactions converting syngas to methanol and, optionally, to higher alcohols and/or other products. Suitable catalysts may include, but are not limited to, one or more of ZnO/Cr₂O₃, Cu/ZnO, Cu/ZnO/Al₂O₃, Cu/ZnO/Cr₂O₃, Cu/ThO₂, Co/Mo/S, Co/S, Mo/S, Ni/S, Ni/Mo/S, Ni/Co/Mo/S, Rh, Ti, Fe, Ir, and any of the foregoing in combination with Mn and/or V. The addition of basic promoters (e.g. K, Li, Na, Rb, Cs, and Fr) increases the activity and selectivity of some of these catalysts for alcohols. Basic promoters include alkaline-earth and rare-earth metals. Non-metallic bases can also serve as effective promoters, in some embodiments.

The catalyst phase can be a packed bed or a fluidized bed. The catalyst particles can be sized and configured such that the chemistry is, in some embodiments, mass-transfer-limited or kinetically limited. The catalyst can take the form of a powder, pellets, granules, beads, extrudates, and so on. When a catalyst support is optionally employed, the support may assume any physical form such as pellets, spheres, monolithic channels, etc. The supports may be coprecipitated with active metal species; or the support may be treated with the catalytic metal species and then used as is or formed into the aforementioned shapes; or the support may be formed into the aforementioned shapes and then treated with the catalytic species.

Up to 50% of CO in syngas feedstream **100** is
converted to methanol in reaction zone **110.** Intermediate product stream **115** output from reactor **105** may comprise, in some variations, 5% to 50% methanol, 5% to 50%> ethanol, 5% to 25% CO, 5% to 25% H₂, and 2% to 35% CO₂, as well as other gases. In some embodiments, intermediate product stream **115** also comprises one or more higher alcohols, such as ethanol, propanol, or butanol.

The H₂/CO ratio in intermediate product stream **115** can, in some variations, affect the yield of ethanol and other products in reactor **120.** The preferred H₂/CO ratio in such variations may depend on the catalyst or catalysts used in reactor **120** as well as on the operating conditions. The H₂/CO ratio in intermediate product stream **115** can differ from that of feedstream **100** as a result of reactions occurring in reactor **105.** In some variations, the H₂/CO ratio of intermediate product stream **115** provides a higher ethanol yield in reactor **120** than would the H₂/CO ratio of feedstream **100.** In such variations, operation of reactor **105** to produce methanol, for example, improves the Hᵥ/CO ratio of the syngas fed to reactor **120** from the standpoint of ethanol yield in reactor **120.**

Feedstream **100** comprises syngas with an H₂/CO ratio of 1.5 to 2, and the preferred H₂/CO ratio for production of ethanol in reactor **120** is about 1. Operation of reactor **105** to produce methanol in this example depletes H₂ in the syngas to decreases the H₂/CO ratio in intermediate product stream **115** to a value closer to 1 and thus improves the ethanol yield in reactor **120.** In certain embodiments, the catalyst in reactor **105** is a Cu/ZnO/alumina catalyst.

Reactor **120** may be any type of catalytic reactor suitable for the conversion of syngas, methanol, and/or syngas plus methanol to ethanol and, optionally, to higher alcohols and/or other products. Reactor **120** may be any suitable fixed-bed reactor, for example. In some variations, reactor **120** comprises tubes filled with one or more catalysts. Syngas and/or methanol passing through the tubes undergoes surface catalyzed reactions to form ethanol and, in some variations, higher alcohols and/or other products.

While not intending to be bound by any particular theory, it is presently believed that the methanol may be converted to syngas and thence to ethanol, the methanol may be converted directly to ethanol via a homologation reaction, and/or the methanol may be converted to ethanol by other mechanisms.

Reactor **120** may operate, for example, at temperatures of 260°C (500°F) to 427°C (800°F) and at pressures of 34.47 x 10⁵ Pag (500 psig) to 172.37 x 10⁵ Pag (2500 psig). In some embodiments, the temperature is between 260°C (500°F) to 316°C (600°F), 316°C (600°F) to 371°C (700°F), or 371°C (700°F) to 427°C (800°F). In some embodiments, the pressure is
34.47 x 10⁵ Pag (500 psig) to 68.95 x 10⁵ Pag (1000 psig), 68.95 x 10⁵ Pag (1000 psig) to 137.90 x 10⁵ Pag (2000 psig), or 137.90 x 10⁵ Pag (2000 psig) to 172.37 x 10⁵ Pag (2500 psig).

Any suitable catalyst or combination of catalysts may be used in reactor **120** to catalyze reactions converting syngas, methanol, and/or syngas + methanol to ethanol and, optionally, to higher alcohols and/or other products. Suitable catalysts may include, but are not limited to, alkali/ZnO/Cr₂O₃, Cu/ZnO, Cu/ZnO/Al₂O₃, CuO/CoO, CuO/CoO/Al₂O₃, Mo/S, Co/Mo/S, Ni/S, Ni/Mo/S, Ni/Co/Mo/S, Rh/Ti/SiO₂, Rh/Mn/SiO₂, Rh/Ti/Fe/Ir/SiO₂, Rh/Mn/MCM-41, Cu, Zn, Rh, Ti, Fe, Ir, and mixtures thereof. The addition of basic promoters (e.g. K, Li, Na, Rb, Cs, and Fr) increases the activity and selectivity of some of these catalysts for ethanol or other C₂+ alcohols. Basic promoters include alkaline-earth and rare-earth metals. Non-metallic bases can also serve as effective promoters, in some embodiments.

In some embodiments, catalysts for reactor **120** can include one or more of ZnO/Cr₂O₃, Cu/ZnO, Cu/ZnO/Al₂O₃, CuO/CoO, CuO/CoO/Al₂O₃, Co/S, Mo/S, Co/Mo/S, Rh/Ti/SiO₂, Rh/Mn/SiO₂, Rh/Ti/Fe/Ir/SiO₂, Rh/Mn/MCM-41, Ni/S, Ni/Mo/S, Ni/Co/Mo/S, and any of the foregoing in combination with Mn and/or V. Again, any of these catalysts can (but do not necessarily) include one or more basic promoters.

The composition of catalysts in reactors **105** and **120,** or reaction zones **110** and **125,** can be similar or even the same. Reference to a "first catalyst" and "second catalyst" in conjunction with reaction zones is a reference to different physical materials, not necessarily a reference to different catalyst compositions. In some embodiments, a certain type of catalyst is loaded into both reaction zones but, over time, the nominal composition of these catalysts could diverge somewhat due to different exposure conditions.

Product stream **130** output from reactor **120** may comprise, in some variations, 0% to 50% methanol, 10% to 90% ethanol, 0% to 25% CO, 0% to 25% H₂, and 5% to 25% CO₂, as well as other gases. In some embodiments, product stream 130 also comprises one or more higher alcohols, such as propanol or butanol.

Referring again to FIG. 1, in some variations unreacted syngas in product stream **130** is separated from product stream **130** to form feedstream **135** and recycled through reactor **120** to further increase, for example, the yield of ethanol and/or other desired products. Alternatively, or in addition, in some variations unreacted syngas in product stream **130** is recycled through reactor **105** by adding it to syngas feedstream **100.** The latter approach may be unsuitable, however, if the unreacted syngas in product stream **130** is contaminated, for example, with sulfur, sulfur compounds, metals, or other materials that can poison methanol catalysts in reactor **105.**

Also, in some variations a methanol feedstream **140** is added to intermediate product stream **115** or otherwise introduced to reactor **120** to further increase, for example, the yield of ethanol and/or other desired products. For example, methanol in product stream **130** may be separated (not shown) from product stream **130** to form feedstream **140** and then recycled through reactor **120.** Methanol from other sources may be introduced, as well or instead, into reactor **120.**

In some variations, one or more catalysts in reactor **105,** one or more catalysts in reactor **120,** or one or more catalysts in both reactor **105** and reactor **120** catalyze the conversion of CO₂ to methanol. Production of methanol in reactor **105,** reactor **120,** or in both reactors may be thereby enhanced by consumption of CO₂ present in syngas feedstream **100.** Consequently, in some variations, CO₂ is added to syngas feedstream **100** or the production and/or subsequent conditioning of syngas feedstream **100** is controlled to produce syngas having a desirable amount of CO₂. Suitable catalysts for converting CO₂ to methanol may include, in some variations, one or more of those listed above for use in reactor **105** and reactor **120.** Enhanced production of methanol by consumption of CO₂ may result, in some variations, in enhanced production of ethanol by conversion of the methanol to ethanol and/or by a resulting favorable adjustment of the H₂/CO ratio in the syngas stream introduced to reactor **120.**

Referring now to FIG. 2, some alternative variations differ from those described above primarily by use of a single reactor **200** comprising a first reaction zone **205** and a second reaction zone **810** rather than two reactors. Syngas feedstream **100** is introduced into first reaction zone **205,** wherein one or more catalysts convert at least a portion of syngas feedstream **100** to methanol to provide intermediate product stream **115** comprising at least a portion of the unreacted syngas from feedstream **100,** methanol, and, in some variations, higher alcohols and/or other reaction products. At least a portion of intermediate product stream **115** is introduced into second reaction zone **810,** where one or more catalysts convert at least a portion of syngas from intermediate product stream **115** and/or at least a portion of methanol from intermediate product stream **115** to provide product stream **130** comprising ethanol and, in some variations, methanol, higher alcohols, other reaction products, and/or unreacted syngas from intermediate product stream **115.**

Reactor **200** may be any type of suitable catalytic reactor comprising two or more reaction zones. Operation of reactor **200** may be similar to the operation of reactors **105** and **120** described above. In particular, in some variations, the catalysts used in reactions zones **205** and **810** and the operating conditions for the reaction zones arc the same as or similar to those for, respectively, reaction zones **110** and **120** described above. The compositions of intermediate product stream **115** and product stream **130** may, in some variations, be the same as or similar to those for the variations described above with respect to FIG. 1. Syngas in product stream **130** may be recycled through reaction zone **810** or added to feedstream **100.** CO₂ may be added to syngas feedstream **100** or the production and/or subsequent conditioning of syngas feedstream **100** may be controlled to produce syngas having a desirable amount of CO₂ for enhanced methanol production. A methanol feedstream (not shown) may be introduced to reaction zone **810** to further increase, for example, the yield of ethanol and/or other desired products. This methanol feedstream may be separated from product stream **130,** for example.

Similarly to the two-reactor variations, in some of the single-reactor variations the H₂/CO ratio in intermediate product stream **115** can affect the yield of ethanol and other products in reaction zone **810.** In some variations, the H₂/CO ratio of intermediate product stream **115** differs from that of feedstream **100** and provides a higher ethanol yield in reaction zone **810** than would the H₂/CO ratio of feedstream **100.** In such variations, production of methanol in reaction zone **205,** for example, improves the H₂/CO ratio of the syngas fed to reaction zone **810** from the standpoint of ethanol yield in reactor **120.**

Referring now to FIG. 3, some alternative variations differ from those described with respect to FIG. 1 in that at least a portion (some or substantially all) of the methanol in intermediate product stream **115** is diverted into a methanol product stream **300** prior to the introduction of product stream **115** into reactor **120.** Methanol in product stream **300** can be separated and purified by conventional methods. Similarly as above, in some of these variations, the H₂/CO ratio of intermediate product stream **115** differs from that of feedstream **100** and provides a higher ethanol yield in reactor **120** than would the H₂/CO ratio of feedstream **100.** Hence, the production of methanol in reactor **105** may advantageously enhance ethanol production in reactor **120** in some of these variations.

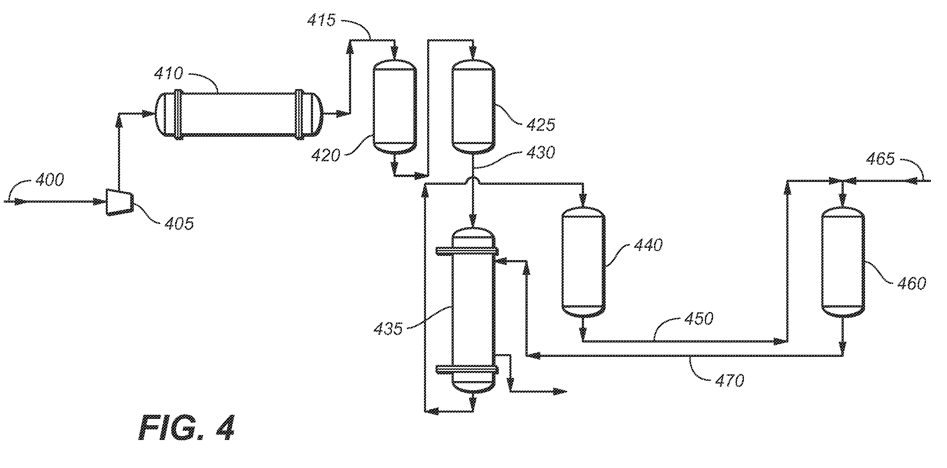

In some variations methanol is produced at high yield in a first reactor and subsequently converted to ethanol in a second reactor. One example is described with reference to FIG. 4 described in more detail below.

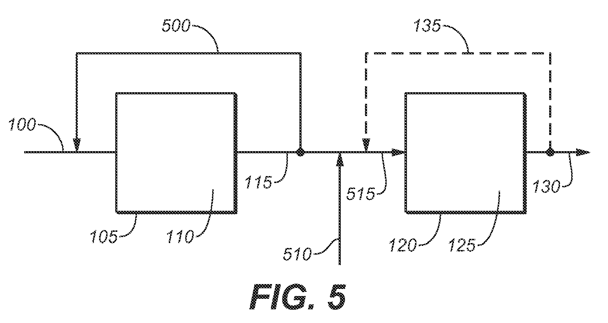

Referring to FIG. 5, for example, in some variations a syngas feedstream **100** is catalytically converted to methanol in a first reactor **105** at a yield (mole conversion of CO to methanol) of, for example, at least 50%, preferably at least 75% or even higher. Such high methanol yields may be facilitated, for example, by separating out some or substantially all of the non-methanol components in intermediate product stream **115** as a stream **500** that is recycled through reactor **105.**

An unrecycled portion of intermediate product stream **115,** rich in methanol, is (optionally) mixed with another syngas feedstream **510** to provide feedstream **515** which is introduced into reactor **120.** At least a portion of the methanol and (optionally) syngas introduced into reactor **120** is catalytically converted to provide a product stream **130** comprising ethanol and, in some variations, methanol, higher alcohol, other reaction products, and/or unreacted syngas from feedstream **515.** In some variations, unreacted syngas in product stream **130** is recycled through reactor **120** as feedstream **135** and/or recycled through reactor **105.** Various components of product stream **130** may be separated out and/or purified as described above.

In some variations, the ratio of methanol to CO in feedstream **100** may be adjusted, for example, to optimize the yield of ethanol in reactor **120.** In some embodiments, the molar ratio of methanol/CO in reactor **120** is between 0.5 to 2.0. In particular embodiments, the ratio of methanol/CO in reactor **120** is 1.0.

Any suitable catalyst or combination of catalysts may be used in reactor **105.** Suitable catalysts for reactor **105** may include, but are not limited to, the methanol catalysts listed above. Similarly, any suitable catalyst or combination of catalysts may be used in reactor **120.** Suitable catalysts for reactor **120** may include, but are not limited to, the ethanol catalysts listed above. The composition of catalysts in reactors **105** and **120** can be similar or even substantially the same.

In variations of any of the methods described herein that use a first reaction zone and a second reaction zone, the initial syngas stream can be introduced into both the first reaction zone and the second reaction zone. In some embodiments, the syngas is from an external source. In some embodiments, the syngas is from any of the methods described herein (such as residual syngas from a first reaction zone or a second reaction zone).

In some embodiments of any of the methods described herein, syngas from any source is added to the first reaction zone and/or the second reaction zone. In some embodiments of any of the methods described herein, methanol from any source is added to the second reaction zone.

Certain embodiments employ a plurality of physical reactors in one or both of the reaction zones. For example, the first zone could consist of two reactors, followed by a single reactor as the second zone. Or, in another example, the first zone could be one reactor followed by two reactors in the second zone. In general, any "zone" or "reaction zone" can contain a fraction of one, two, three, or more physical reactors.

In some embodiments of any of the methods described herein, reaction conditions (such as the temperature and pressure) used for the conversion of syngas to methanol, the conversion of syngas and/or methanol to ethanol, or the homologation of methanol to ethanol are the same as those described in any of U.S. Patent Nos. 4,371,724; 4,424,384; 4,374,285; 4,409,405; 4,277,634; 4,253,987; 4,233,466; and 4,171,461.

FIG. 4 shows an example of a process in which syngas is catalytically converted to methanol in a first reactor, and methanol and residual syngas from the first reactor are converted to ethanol in a second reactor. Referring now to FIG. 4, a single two-stage intercooled reciprocating compressor **405** compresses syngas feedstream **400** to 103.41 x 10⁵ Pag (1500 psig) and feeds it at a temperature of 57°C (135°F) to syngas preheater **410.** Preheater **410** is a shell and tube heat exchanger that uses steam as an enthalpy source.

In this example associated with FIG. 4, heated syngas **415** from preheater **410** is sent to a set of reactor guard beds **420, 425.** Guard beds **420, 425** are configured in a permanent lead-lag arrangement but are piped such that either bed can be bypassed. The piping arrangement allows one bed to be in service while the other is being regenerated or activated. Regeneration is initiated by a mixed hydrogen and nitrogen line (not shown). Guard beds **415, 420** remove, for example, sulfurs and metals that may poison the methanol catalysts. In some embodiments, one or more catalyst poisons are removed by adsorption over copper, copper chromite, nickel, cobalt, or molybdenum. These and other metals can be supported on high-surface-area refractory inorganic oxide materials such as alumina, silica, silica/alumina, clays, or kieselguhr. One exemplary material is copper on alumina. Exit gases **430** from guard beds **420, 425** are sent to an alcohol reactor cross exchanger **435** at 177°C (350°F) and are heated to 249°C (480°F) during heat exchange with crude alcohol exit gases 470 from second alcohol reactor **460.**

With continuing reference to FIG. 4, syngas at 103.41 x 10⁵ Pag (1500 psig) and 249°C (480°F) enters a first alcohol synthesis reactor **440,** where at least a portion of the syngas undergoes a catalyzed reaction in supported-catalyst tubular reactors within the reactor vessel. In some variations, the catalyst in reactor **440** is a Cu/ZnO/alumina catalyst. Methanol is expected to be formed via the reaction CO + 2 H₂ > CH₃OH. As noted earlier in this detailed description, in some variations methanol may be formed by the hydrogenation of CO₂ as well.

Product gases **450** leave alcohol synthesis reactor **440** at a temperature of 260°C (500°F) and enter alcohol synthesis reactor **460.** In addition, a methanol stream **465** (e.g., a methanol recycle stream separated from crude alcohol stream **470**)
is mixed with the product gases **450** from reactor **440** and also introduced to reactor **460.** Reactions occurring in reactor **460** can include ethanol formation.

Crude alcohol stream **470** exits reactor **460** at a temperature of 343°C (650°F) and is cooled by heat exchange in alcohol reactor cross exchanger **435** to a temperature of 277°C (530°F). Subsequent heat recovery and other cooling steps (not shown) cool crude alcohol stream **470** to 38°C (100°F). Ethanol, methanol, residual syngas, and other components of crude alcohol stream **470** may be separated and (optionally) purified by using the methods described herein or using conventional methods (not shown). Syngas recovered from stream **470** may, for example, be recycled through the reactors by mixing it with syngas feedstream **400.**

Some variations may employ microwave, radio frequency, laser, and/or UV energy in addition to or instead of conventional process heat (e.g., steam, heat from burners, waste heat, etc.) to facilitate the production of ethanol. For example, microwave, radio frequency, laser, and/or UV energy may be used in some variations to convert CO₂ in syngas to CO and O₂ for more efficient catalytic conversion to methanol and/or ethanol. In some embodiments, a conventional method for converting CO₂ in syngas to CO (e.g., treating syngas with a catalyst that promotes the conversion of CO₂ to CO) is used for more efficient catalytic conversion to methanol and/or ethanol. In some embodiments, both a catalyst and irradiation (such as irradiation with microwave, radio frequency, laser, and/or UV energy) are used to convert CO₂ to CO. In particular embodiments, CO₂ is removed from the syngas and irradiation (such as irradiation with microwave, radio frequency, laser, and/or UV energy) and/or a catalyst (such as a thermal catalyst) is used to generate O₂ from CO. The O₂ is removed and the CO is added to the first and/or second reactor zone. In some embodiments, the irradiation allows a lower temperature and/or pressure to be used for conversion of CO₂ to CO than the standard temperatures and pressures used for conversion of CO₂ to CO without irradiation. CO₂ in syngas stream **100** may be optionally converted in this manner in some variations.

As another example, microwave, radio frequency, laser, and/or UV energy may be used to accelerate the catalytic conversion of syngas to methanol and/or ethanol, and/or to accelerate the catalytic conversion of syngas and/or methanol to ethanol in variations of the processes described above for conversion of
syngas to ethanol. More generally, in some variations, microwave, radio frequency, laser, and/or UV energy may be used to accelerate the catalytic conversion of syngas of any origin to methanol and/or ethanol, and/or to accelerate the catalytic conversion of syngas and/or methanol of any origin to ethanol.

In some embodiments, microwave, radio frequency, laser, and/or UV energy is used to irradiate syngas and/or the first catalyst in the first reaction zone to enhance the conversion of syngas to methanol. In some embodiments, the irradiation increases molecular vibrations, increases the energy density, or otherwise activates the syngas and/or first catalyst. Such use of microwave, radio frequency, laser, and/or UV energy in a syngas-to-methanol reactor, for example, may allow the reactor to be operated at lower temperatures and pressures than otherwise.

In some variations, microwave, radio frequency, laser, and/or UV energy is used to irradiate the syngas, methanol, and/or the second catalyst in the second reaction zone. In some embodiments, the irradiation increases molecular vibrations, increases the energy density, or otherwise activates the syngas, methanol, and/or second catalyst. Enhancement of catalytic conversion of methanol to ethanol may occur, for example, by preferential absorption of the microwave, radio frequency, laser, and/or UV energy by the methanol allowing high energy densities to be achieved in the methanol reactants. For example, microwaves heat methanol at a faster rate than ethanol, thereby favoring the conversion of methanol to ethanol. Such use of microwave, radio frequency, laser, and/or UV energy in a methanol to ethanol reactor, for example, may allow the reactor to be operated at lower temperatures and pressures than otherwise.

In some embodiments, methods involve introducing syngas into a reaction zone (e.g., a reactor) comprising at least one catalyst, and irradiating the syngas and/or the catalyst in the reaction zone with energy (e.g., microwave, radio frequency, laser, and/or UV energy). At least a portion of the syngas can be converted to ethanol. The method may also produce methanol or other alcohols. Suitable catalysts may include, but are not limited to, any of the catalysts described herein. In some embodiments, the catalyst is a conventional catalyst for the conversion of syngas to ethanol in one reaction zone or one reactor. In some embodiments, the catalyst favors the formation of ethanol over methanol in the absence of irradiation, and the irradiation enhances the selectivity for the formation of ethanol. For example, the irradiation may heat methanol at a faster rate than ethanol, thereby favoring the conversion of methanol to ethanol. In some embodiments, the catalyst favors the formation of methanol over ethanol in the absence of irradiation, and the irradiation causes the catalyst to produce a lower ratio of methanol to ethanol than in the absence of irradiation. For example, irradiation may cause the catalyst to now produce more ethanol than methanol.

## Claims

1. A method of producing one or more C₂-C₄ alcohols, said method comprising:
(i) introducing syngas into a first reaction zone comprising at least a first catalyst;
(ii) converting a portion of up to 50% CO of said syngas to methanol with said first catalyst;
(iii)introducing syngas and methanol from said first reaction zone into a second reaction zone comprising at least a second catalyst; and
(iv) converting at least a portion of said syngas and methanol introduced into said second reaction zone with said second catalyst to produce a product stream comprising one or more C₂-C₄ alcohols selected from ethanol, propanol and butanol;
wherein said first catalyst and said second catalyst are different physical materials; and
wherein said said syngas introduced into said first reaction zone has an initial H₂/CO ratio of 1.5 to 2, conversion of syngas to methanol in said first reaction zone causes said syngas introduced into said second reaction zone to have a second H₂/CO ratio which is lower than said initial H₂/CO ratio, and said second H₂/CO ratio provides an increased yield to one or more C₂-C₄ alcohols in said second reaction zone compared to that which would be provided by said initial H₂/CO ratio.

2. The method of claim 1, wherein said C₂-C₄ alcohols comprise ethanol.

3. The method of claim 1, further comprising introducing additional methanol into said second reaction zone and converting at least a portion of said additional methanol introduced into said second reaction zone to one or more C₂-C₄ alcohols with said second catalyst.

## Patentansprüche

1. Verfahren zur Herstellung eines oder mehrerer C₂-C₄-Alkohole, welches Verfahren umfasst:
(i) Einführung von Syngas in einer erste Reaktionszone, die mindestens einen ersten Katalysator umfasst;
(ii) Umwandlung eines Teils von bis zu 50% CO des Syngases mit dem ersten Katalysator in Methanol;
(iii) Einführung von Syngas und Methanol aus der ersten Reaktionszone in a zweite Reaktionszone, die mindestens einen zweiten Katalysator umfasst; und
(iv) Umwandlung mindestens eines Teils des in die zweite Reaktionszone eingeführten Syngases und Methanols mit dem zweiten Katalysator, um einen Produktstrom zu erzeugen, der einen oder mehrere C₂-C₄-Alkohole ausgewählt aus Ethanol, Propanol und Butanol umfasst;
wobei der erste Katalysator und der zweite Katalysator verschiedene physikalische Materialien sind; und
wobei das in die erste Reaktionszone eingeführte Syngas ein anfängliches H₂/CO-Verhältnis von 1,5 bis 2 aufweist, die Umwandlung von Syngas in Methanol in der ersten Reaktionszone das in die zweite Reaktionszone eingeführte Syngas dazu bringt, ein zweites H₂/CO-Verhältnis zu haben, das niedriger ist als das anfängliche H₂/CO-Verhältnis, und das zweite H₂/CO-Verhältnis eine erhöhte Ausbeute an einem oder mehreren C₂-C₄-Alkoholen in der zweiten Reaktionszone liefert, verglichen mit demjenigen das durch das anfängliche H₂/CO-Verhältnis erhalten würde.

2. Verfahren nach Anspruch 1, bei dem die C₂-C₄-Alkohole Ethanol umfassen.

3. Verfahren nach Anspruch 1, das ferner die Einführung von zusätzlichem Methanol in die zweite Reaktionszone und Umwandlung von mindestens einem Teil des in die zweite Reaktionszone eingeführten zusätzlichen Methanols mit dem zweiten Katalysator in einen oder mehrere C₂-C₄-Alkohole umfasst.

## Revendications

1. Procédé de production d'un ou plusieurs alcools en C₂ à C₄, ledit procédé comprenant :
(i) l'introduction d'un gaz de synthèse dans une première zone de réaction comprenant au moins un premier catalyseur;
(ii) la conversion d'une partie pouvant atteindre 50 % de CO dudit gaz de synthèse en méthanol avec ledit premier catalyseur ;
(iii) l'introduction du gaz de synthèse et du méthanol provenant de ladite première zone de réaction dans une deuxième zone de réaction comprenant au moins un deuxième catalyseur ; et
(iv) la conversion d'au moins une partie dudit gaz de synthèse et dudit méthanol introduits dans ladite deuxième zone de réaction avec ledit deuxième catalyseur pour produire un courant de produit comprenant un ou plusieurs alcools en C₂ à C₄ choisis parmi l'éthanol, le propanol et le butanol ;
dans lequel ledit premier catalyseur et ledit deuxième catalyseur sont des matériaux physiques différents ; et
dans lequel ledit gaz de synthèse introduit dans ladite première zone de réaction a un rapport H₂/CO initial de 1,5 à 2, la conversion du gaz de synthèse en méthanol dans ladite première zone de réaction confère audit gaz de synthèse introduit dans ladite deuxième zone de réaction un deuxième rapport H₂/CO qui est inférieur audit rapport H₂/CO initial, et ledit deuxième rapport H₂/CO confère un rendement accru à un ou plusieurs alcools en C₂ à C₄ dans ladite deuxième zone de réaction en comparaison avec celui qui serait conféré par ledit rapport H₂/CO initial.

2. Procédé selon la revendication 1, dans lequel lesdits alcools en C₂ à C₄ comprennent de l'éthanol.

3. Procédé selon la revendication 1, comprenant en outre l'introduction de méthanol additionnel dans ladite deuxième zone de réaction et la conversion d'au moins une partie dudit méthanol additionnel introduit dans ladite deuxième zone de réaction en un ou plusieurs alcools en C₂ à C₄ avec ledit deuxième catalyseur.
